# EUROPEAN PATENT APPLICATION

(11) **EP 0 753 527 A1**
(43) Date of publication of application: **15.01.1997**
(21) Application number: 95913316.6
(22) Date of filing: 22.03.1995
(51) Int. Cl.: C07K 14/705, C12N 15/00, C12N 5/00, C12P 21/02, C07K 16/28, C12P 21/08, A61K 38/17, G01N 33/566, A61K 39/395

(54) **PROSTAGLANDIN E RECEPTOR**

(30) Priority: 23.03.1994 JP 76716/94
(71) Applicant: ONO PHARMACEUTICAL CO., LTD., Osaka-shi Osaka 541 (JP)
(72) Inventor: ICHIKAWA, Atsushi, Takatsuki-shi, Osaka 569 (JP); NAKAO, Kazuwa, Kyoto-shi, Kyoto 610-11 (JP); NARUMIYA, Shuh, Kyoto-shi, Kyoto 610-11 (JP)
(74) Representative: Bentham, Stephen
(86) International application number: JP9500522
(87) International publication number: WO9525747

(57) **Abstract**

A polypeptide of human and rat EP2 receptors; a process for producing the same; a DNA coding for the same; a replication or expression vector comprising the DNA and a host cell transformed by the vector; a monoclonal or polyclonal antibody of the polypeptide; a pharmaceutical composition containing the polypeptide or the antibody; and a method of screening a compound having an EP2 agonist or antagonist activity by using the polypeptide and the host cell. As the above polypeptide itself specifically binds to PGE₂, it can be used as a preventive or remedy for diseases caused by PGE₂ overproduction, such as inflammation. Also it can be used for screening a substance having an EP2 receptor agonist or antagonist activity.

(1) GENERAL INFORMATION:

(2) INFORMATION FOR SEQ ID NO: 1:

(i) SEQUENCE CHARACTERISTICS:
(A) LENGTH 488 amino acids
(B) TYPE: amino acid
(D) TOPOLOGY: linear
(ii) MOLECULE TYPE: protein

(xi) SEQUENCE DESCRIPTION: SEQ ID NO: 1

Met Ser Thr Pro Gly Val Asn Ser Ser Ala Ser Leu Ser Pro Asp Arg
1               5                   10                  15
Leu Asn Ser Pro Val Thr Ile Pro Ala Val Met Phe Ile Phe Gly Val
            20                  25                  30
Val Gly Asn Leu Val Ala Ile Val Val Leu Cys Lys Ser Arg Lys Glu
        35                  40                  45
Gln Lys Glu Thr Thr Phe Tyr Thr Leu Val Cys Gly Leu Ala Val Thr
    50                  55                  60
Asp Leu Leu Gly Thr Leu Leu Val Ser Pro Val Thr Ile Ala Thr Tyr
65                  70                  75                  80
Met Lys Gly Gln Trp Pro Gly Gly Gln Pro Leu Cys Glu Tyr Ser Thr
                85                  90                  95
Phe Ile Leu Leu Phe Phe Ser Leu Ser Gly Leu Ser Ile Ile Cys Ala
            100                 105                 110
Met Ser Val Glu Arg Tyr Leu Ala Ile Asn His Ala Tyr Phe Tyr Ser
        115                 120                 125
His Tyr Val Asp Lys Arg Leu Ala Gly Leu Thr Leu Phe Ala Val Tyr
    130                 135                 140
Ala Ser Asn Val Leu Phe Cys Ala Leu Pro Asn Met Gly Leu Gly Ser
145                 150                 155                 160
Ser Arg Leu Gln Tyr Pro Asp Thr Trp Cys Phe Ile Asp Trp Thr Thr
                165                 170                 175
Asn Val Thr Ala His Ala Ala Tyr Ser Tyr Met Tyr Ala Gly Phe Ser
            180                 185                 190
Ser Phe Leu Ile Leu Ala Thr Val Leu Cys Asn Val Leu Val Cys Gly
        195                 200                 205
Ala Leu Leu Arg Met His Arg Gln Phe Met Arg Arg Thr Ser Leu Gly
    210                 215                 220
Thr Glu Gln His His Ala Ala Ala Ala Ala Ser Val Ala Ser Arg Gly
225                 230                 235                 240
His Pro Ala Ala Ser Pro Ala Leu Pro Arg Leu Ser Asp Phe Arg Arg
                245                 250                 255
Arg Arg Ser Phe Arg Arg Ile Ala Gly Ala Glu Ile Gln Met Val Ile
            260                 265                 270
Leu Leu Ile Ala Thr Ser Leu Val Val Leu Ile Cys Ser Ile Pro Leu
        275                 280                 285
Val Val Arg Val Phe Val Asn Gln Leu Tyr Gln Pro Ser Leu Glu Arg
    290                 295                 300
Glu Val Ser Lys Asn Pro Asp Leu Gln Ala Ile Arg Ile Ala Ser Val
305                 310                 315                 320
Asn Pro Ile Leu Asp Pro Trp Ile Tyr Ile Leu Leu Arg Lys Thr Val
                325                 330                 335
Leu Ser Lys Ala Ile Glu Lys Ile Lys Cys Leu Phe Cys Arg Ile Gly
            340                 345                 350

Gly Ser Arg Arg Glu Arg Ser Gly Gln His Cys Ser Asp Ser Gln Arg
        355                 360                 365
Thr Ser Ser Ala Met Ser Gly His Ser Arg Ser Phe Ile Ser Arg Glu
    370                 375                 380
Leu Lys Glu Ile Ser Ser Thr Ser Gln Thr Leu Leu Pro Asp Leu Ser
385                 390                 395                 400
Leu Pro Asp Leu Ser Glu Asn Gly Leu Gly Gly Arg Asn Leu Leu Pro
                405                 410                 415
Gly Val Pro Gly Met Gly Leu Ala Gln Glu Asp Thr Thr Ser Leu Arg
            420                 425                 430
Thr Leu Arg Ile Ser Glu Thr Ser Asp Ser Ser Gln Gly Gln Asp Ser
        435                 440                 445
Glu Ser Val Leu Leu Val Asp Glu Ala Gly Gly Ser Gly Arg Ala Gly
    450                 455                 460
Pro Ala Pro Lys Gly Ser Ser Leu Gln Val Thr Phe Pro Ser Glu Thr
465                 470                 475                 480
Leu Asn Leu Ser Glu Lys Cys Ile
                485

(2) INFORMATION FOR SEQ ID NO: 2:

    (i) SEQUENCE CHARACTERISTICS:
        (A) LENGTH 1464 base pairs
        (B) TYPE: nucleic acid
        (C) STRANDEDNESS: single
        (D) TOPOLOGY: linear
    (ii) MOLECULE TYPE: cDNA to mRNA

    (xi) SEQUENCE DESCRIPTION: SEQ ID NO: 2

ATGTCCACTC CCGGGGTCAA TTCGTCCGCC TCCTTGAGCC CCGACCGGCT GAACAGCCCA   60
GTGACCATCC CGGCGGTGAT GTTCATCTTC GGGGTGGTGG GCAACCTGGT GGCCATCGTG  120
GTGCTGTGCA AGTCGCGCAA GGAGCAGAAG GAGACGACCT TCTACACGCT GGTATGTGGG  180
CTGGCTGTCA CCGACCTGTT GGGCACTTTG TTGGTGAGCC CGGTGACCAT CGCCACGTAC  240
ATGAAGGGCC AATGGCCCGG GGGCCAGCCG CTGTGCGAGT ACAGCACCTT CATTCTGCTC  300
TTCTTCAGCC TGTCCGGCCT CAGCATCATC TGCGCCATGA GTGTCGAGCG CTACCTGGCC  360
ATCAACCATG CCTATTTCTA CAGCCACTAC GTGGACAAGC GATTGGCGGG CCTCACGCTC  420
TTTGCAGTCT ATGCGTCCAA CGTGCTCTTT TGCGCGCTGC CCAACATGGG TCTCGGTAGC  480
TCGCGGCTGC AGTACCCAGA CACCTGGTGC TTCATCGACT GGACCACCAA CGTGACGGCG  540
CACGCCGCCT ACTCCTACAT GTACGCGGGC TTCAGCTCCT TCCTCATTCT CGCCACCGTC  600
CTCTGCAACG TGCTTGTGTG CGGCGCGCTG CTCCGCATGC ACCGCCAGTT CATGCGCCGC  660
ACCTCGCTGG GCACCGAGCA GCACCACGCG GCCGCGGCCG CCTCGGTTGC CTCCCGGGGC  720
CACCCCGCTG CCTCCCCAGC CTTGCCGCGC CTCAGCGACT TTCGGCGCCG CCGGAGCTTC  780
CGCCGCATCG CGGGCGCCGA GATCCAGATG GTCATCTTAC TCATTGCCAC CTCCCTGGTG  840
GTGCTCATCT GCTCCATCCC GCTCGTGGTG CGAGTATTCG TCAACCAGTT ATATCAGCCA  900
AGTTTGGAGC GAGAAGTCAG TAAAAATCCA GATTTGCAGG CCATCCGAAT TGCTTCTGTG  960
AACCCCATCC TAGACCCCTG GATATATATC CTCCTGAGAA AGACAGTGCT CAGTAAAGCA 1020
ATAGAGAAGA TCAAATGCCT CTTCTGCCGC ATTGGCGGGT CCCGCAGGGA GCGCTCCGGA 1080
CAGCACTGCT CAGACAGTCA AAGGACATCT TCTGCCATGT CAGGCCACTC TCGCTCCTTC 1140
ATCTCCCGGG AGCTGAAGGA GATCAGCAGT ACATCTCAGA CCCTCCTGCC AGACCTCTCA 1200
CTGCCAGACC TCAGTGAAAA TGGCCTTGGA GGCAGGAATT TGCTTCCAGG TGTGCCTGGC 1260
ATGGGCCTGG CCCAGGAAGA CACCACCTCA CTGAGGACTT TGCGAATATC AGAGACCTCA 1320
GACTCTTCAC AGGGTCAGGA CTCAGAGAGT GTCTTACTGG TGGATGAGGC TGGTGGGAGC 1380
GGCAGGGCTG GGCCTGCCCC TAAGGGGAGC TCCCTGCAAG TCACATTTCC CAGTGAAACA 1440
CTGAACTTAT CAGAAAAATG TATA                                       1464

(2) INFORMATION FOR SEQ ID NO: 3:

(i) SEQUENCE CHARACTERISTICS:
(A) LENGTH 1554 base pairs
(B) TYPE: nucleic acid
(C) STRANDEDNESS: single
(D) TOPOLOGY: linear
(ii) MOLECULE TYPE: cDNA to mRNA

(xi) SEQUENCE DESCRIPTION: SEQ ID NO: 3

CCTGGGGGCT CGTGAGGCTG CCACCGCTGC TGCCGCTACA GACCCAGCCT TGCACTCCAA 60
GGCTGCGCAC CGCCAGCCAC TATCATGTCC ACTCCCGGGG TCAATTCGTC CGCCTCCTTG 120
AGCCCCGACC GGCTGAACAG CCCAGTGACC ATCCCGGCGG TGATGTTCAT CTTCGGGGTG 180
GTGGGCAACC TGGTGGCCAT CGTGGTGCTG TGCAAGTCGC GCAAGGAGCA GAAGGAGACG 240
ACCTTCTACA CGCTGGTATG TGGGCTGGCT GTCACCGACC TGTTGGGCAC TTTGTTGGTG 300
AGCCCGGTGA CCATCGCCAC GTACATGAAG GGCCAATGGC CCGGGGGCCA GCCGCTGTGC 360
GAGTACAGCA CCTTCATTCT GCTCTTCTTC AGCCTGTCCG GCCTCAGCAT CATCTGCGCC 420
ATGAGTGTCG AGCGCTACCT GGCCATCAAC CATGCCTATT TCTACAGCCA CTACGTGGAC 480
AAGCGATTGG CGGGCCTCAC GCTCTTTGCA GTCTATGCGT CCAACGTGCT CTTTTGCGCG 540
CTGCCCAACA TGGGTCTCGG TAGCTCGCGG CTGCAGTACC CAGACACCTG GTGCTTCATC 600
GACTGGACCA CCAACGTGAC GGCGCACGCC GCCTACTCCT ACATGTACGC GGGCTTCAGC 660
TCCTTCCTCA TTCTCGCCAC CGTCCTCTGC AACGTGCTTG TGTGCGGCGC GCTGCTCCGC 720
ATGCACCGCC AGTTCATGCG CCGCACCTCG CTGGGCACCG AGCAGCACCA CGCGGCCGCG 780
GCCGCCTCGG TTGCCTCCCG GGGCCACCCC GCTGCCTCCC CAGCCTTGCC GCGCCTCAGC 840
GACTTTCGGC GCCGCCGGAG CTTCCGCCGC ATCGCGGGCG CCGAGATCCA GATGGTCATC 900
TTACTCATTG CCACCTCCCT GGTGGTGCTC ATCTGCTCCA TCCCGCTCGT GGTGCGAGTA 960
TTCGTCAACC AGTTATATCA GCCAAGTTTG GAGCGAGAAG TCAGTAAAAA TCCAGATTTG 1020
CAGGCCATCC GAATTGCTTC TGTGAACCCC ATCCTAGACC CCTGGATATA TATCCTCCTG 1080
AGAAAGACAG TGCTCAGTAA AGCAATAGAG AAGATCAAAT GCCTCTTCTG CCGCATTGGC 1140
GGGTCCCGCA GGGAGCGCTC CGGACAGCAC TGCTCAGACA GTCAAAGGAC ATCTTCTGCC 1200
ATGTCAGGCC ACTCTCGCTC CTTCATCTCC CGGGAGCTGA AGGAGATCAG CAGTACATCT 1260
CAGACCCTCC TGCCAGACCT CTCACTGCCA GACCTCAGTG AAAATGGCCT TGGAGGCAGG 1320
AATTTGCTTC CAGGTGTGCC TGGCATGGGC CTGGCCCAGG AAGACACCAC CTCACTGAGG 1380
ACTTTGCGAA TATCAGAGAC CTCAGACTCT TCACAGGGTC AGGACTCAGA GAGTGTCTTA 1440
CTGGTGGATG AGGCTGGTGG GAGCGGCAGG GCTGGGCCTG CCCCTAAGGG GAGCTCCCTG 1500
CAAGTCACAT TTCCCAGTGA AACACTGAAC TTATCAGAAA AATGTATATA ATAG 1554

(2) INFORMATION FOR SEQ ID NO: 4:

(i) SEQUENCE CHARACTERISTICS:
(A) LENGTH 1554 base pairs
(B) TYPE: nucleic acid
(C) STRANDEDNESS: single
(D) TOPOLOGY: linear
(ii) MOLECULE TYPE: cDNA to mRNA
(vi) ORIGINAL SOURCE
(A) ORGANISM: Homo sapiens
(F) TISSUE TYPE: lung
(ix) FEATURE
(A) NAME/KEY: CDP
(B) LOCATION: 85..1551
(C) IDENTIFICATION METHOD: P

(xi) SEQUENCE DESCRIPTION: SEQ ID NO: 4

CCTGGGGGCT CGTGAGGCTG CCACCGCTGC TGCCGCTACA GACCCAGCCT TGCACTCCAA 60
GGCTGCGCAC CGCCAGCCAC TATC ATG TCC ACT CCC GGG GTC AAT TCG TCC 111
Met Ser Thr Pro Gly Val Asn Ser Ser
1               5
GCC TCC TTG AGC CCC GAC CGG CTG AAC AGC CCA GTG ACC ATC CCG GCG 159
Ala Ser Leu Ser Pro Asp Arg Leu Asn Ser Pro Val Thr Ile Pro Ala
10                  15                  20                  25
GTG ATG TTC ATC TTC GGG GTG GTG GGC AAC CTG GTG GCC ATC GTG GTG 207
Val Met Phe Ile Phe Gly Val Val Gly Asn Leu Val Ala Ile Val Val
                30                  35                  40
CTG TGC AAG TCG CGC AAG GAG CAG AAG GAG ACG ACC TTC TAC ACG CTG 255
Leu Cys Lys Ser Arg Lys Glu Gln Lys Glu Thr Thr Phe Tyr Thr Leu
            45                  50                  55
GTA TGT GGG CTG GCT GTC ACC GAC CTG TTG GGC ACT TTG TTG GTG AGC 303
Val Cys Gly Leu Ala Val Thr Asp Leu Leu Gly Thr Leu Leu Val Ser
        60                  65                  70
CCG GTG ACC ATC GCC ACG TAC ATG AAG GGC CAA TGG CCC GGG GGC CAG 351
Pro Val Thr Ile Ala Thr Tyr Met Lys Gly Gln Trp Pro Gly Gly Gln
    75                  80                  85
CCG CTG TGC GAG TAC AGC ACC TTC ATT CTG CTC TTC TTC AGC CTG TCC 399
Pro Leu Cys Glu Tyr Ser Thr Phe Ile Leu Leu Phe Phe Ser Leu Ser
90                  95                  100                 105
GGC CTC AGC ATC ATC TGC GCC ATG AGT GTC GAG CGC TAC CTG GCC ATC 447
Gly Leu Ser Ile Ile Cys Ala Met Ser Val Glu Arg Tyr Leu Ala Ile
                110                 115                 120
AAC CAT GCC TAT TTC TAC AGC CAC TAC GTG GAC AAG CGA TTG GCG GGC 495
Asn His Ala Tyr Phe Tyr Ser His Tyr Val Asp Lys Arg Leu Ala Gly
            125                 130                 135
CTC ACG CTC TTT GCA GTC TAT GCG TCC AAC GTG CTC TTT TGC GCG CTG 543
Leu Thr Leu Phe Ala Val Tyr Ala Ser Asn Val Leu Phe Cys Ala Leu
        140                 145                 150
CCC AAC ATG GGT CTC GGT AGC TCG CGG CTG CAG TAC CCA GAC ACC TGG 591
Pro Asn Met Gly Leu Gly Ser Ser Arg Leu Gln Tyr Pro Asp Thr Trp
    155                 160                 165
TGC TTC ATC GAC TGG ACC ACC AAC GTG ACG GCG CAC GCC GCC TAC TCC 639
Cys Phe Ile Asp Trp Thr Thr Asn Val Thr Ala His Ala Ala Tyr Ser
170                 175                 180                 185
TAC ATG TAC GCG GGC TTC AGC TCC TTC CTC ATT CTC GCC ACC GTC CTC 687
Tyr Met Tyr Ala Gly Phe Ser Ser Phe Leu Ile Leu Ala Thr Val Leu
                190                 195                 200
TGC AAC GTG CTT GTG TGC GGC GCG CTG CTC CGC ATG CAC CGC CAG TTC 735
Cys Asn Val Leu Val Cys Gly Ala Leu Leu Arg Met His Arg Gln Phe
            205                 210                 215
ATG CGC CGC ACC TCG CTG GGC ACC GAG CAG CAC CAC GCG GCC GCG GCC 783
Met Arg Arg Thr Ser Leu Gly Thr Glu Gln His His Ala Ala Ala Ala
        220                 225                 230
GCC TCG GTT GCC TCC CGG GGC CAC CCC GCT GCC TCC CCA GCC TTG CCG 831
Ala Ser Val Ala Ser Arg Gly His Pro Ala Ala Ser Pro Ala Leu Pro
    235                 240                 245
CGC CTC AGC GAC TTT CGG CGC CGC CGG AGC TTC CGC CGC ATC GCG GGC 879
Arg Leu Ser Asp Phe Arg Arg Arg Arg Ser Phe Arg Arg Ile Ala Gly
250                 255                 260                 265
GCC GAG ATC CAG ATG GTC ATC TTA CTC ATT GCC ACC TCC CTG GTG GTG 927
Ala Glu Ile Gln Met Val Ile Leu Leu Ile Ala Thr Ser Leu Val Val
                270                 275                 280

CTC ATC TGC TCC ATC CCG CTC GTG GTG CGA GTA TTC GTC AAC CAG TTA 975
Leu Ile Cys Ser Ile Pro Leu Val Val Arg Val Phe Val Asn Gln Leu
            285                 290                 295
TAT CAG CCA AGT TTG GAG CGA GAA GTC AGT AAA AAT CCA GAT TTG CAG 1023
Tyr Gln Pro Ser Leu Glu Arg Glu Val Ser Lys Asn Pro Asp Leu Gln
        300                 305                 310
GCC ATC CGA ATT GCT TCT GTG AAC CCC ATC CTA GAC CCC TGG ATA TAT 1071
Ala Ile Arg Ile Ala Ser Val Asn Pro Ile Leu Asp Pro Trp Ile Tyr
    315                 320                 325
ATC CTC CTG AGA AAG ACA GTG CTC AGT AAA GCA ATA GAG AAG ATC AAA 1119
Ile Leu Leu Arg Lys Thr Val Leu Ser Lys Ala Ile Glu Lys Ile Lys
330                 335                 340                 345
TGC CTC TTC TGC CGC ATT GGC GGG TCC CGC AGG GAG CGC TCC GGA CAG 1167
Cys Leu Phe Cys Arg Ile Gly Gly Ser Arg Arg Glu Arg Ser Gly Gln
                350                 355                 360
CAC TGC TCA GAC AGT CAA AGG ACA TCT TCT GCC ATG TCA GGC CAC TCT 1215
His Cys Ser Asp Ser Gln Arg Thr Ser Ser Ala Met Ser Gly His Ser
            365                 370                 375
CGC TCC TTC ATC TCC CGG GAG CTG AAG GAG ATC AGC AGT ACA TCT CAG 1263
Arg Ser Phe Ile Ser Arg Glu Leu Lys Glu Ile Ser Ser Thr Ser Gln
        380                 385                 390
ACC CTC CTG CCA GAC CTC TCA CTG CCA GAC CTC AGT GAA AAT GGC CTT 1311
Thr Leu Leu Pro Asp Leu Ser Leu Pro Asp Leu Ser Glu Asn Gly Leu
    395                 400                 405
GGA GGC AGG AAT TTG CTT CCA GGT GTG CCT GGC ATG GGC CTG GCC CAG 1359
Gly Gly Arg Asn Leu Leu Pro Gly Val Pro Gly Met Gly Leu Ala Gln
410                 415                 420                 425
GAA GAC ACC ACC TCA CTG AGG ACT TTG CGA ATA TCA GAG ACC TCA GAC 1407
Glu Asp Thr Thr Ser Leu Arg Thr Leu Arg Ile Ser Glu Thr Ser Asp
                430                 435                 440
TCT TCA CAG GGT CAG GAC TCA GAG AGT GTC TTA CTG GTG GAT GAG GCT 1455
Ser Ser Gln Gly Gln Asp Ser Glu Ser Val Leu Leu Val Asp Glu Ala
            445                 450                 455
GGT GGG AGC GGC AGG GCT GGG CCT GCC CCT AAG GGG AGC TCC CTG CAA 1503
Gly Gly Ser Gly Arg Ala Gly Pro Ala Pro Lys Gly Ser Ser Leu Gln
        460                 465                 470
GTC ACA TTT CCC AGT GAA ACA CTG AAC TTA TCA GAA AAA TGT ATA 1548
Val Thr Phe Pro Ser Glu Thr Leu Asn Leu Ser Glu Lys Cys Ile
    475                 480                 485
TAATAG 1554

(2) INFORMATION FOR SEQ ID NO: 5:

(i) SEQUENCE CHARACTERISTICS:
(A) LENGTH 488 amino acids
(B) TYPE: amino acid
(D) TOPOLOGY: linear
(ii) MOLECULE TYPE: protein

(xi) SEQUENCE DESCRIPTION: SEQ ID NO: 5

Met Ser Ile Pro Gly Val Asn Ala Ser Phe Ser Ser Thr Pro Glu Arg
1               5                   10                  15
Leu Asn Ser Pro Val Thr Ile Pro Ala Val Met Phe Ile Phe Gly Val
            20                  25                  30
Val Gly Asn Leu Val Ala Ile Val Val Leu Cys Lys Ser Arg Lys Glu
        35                  40                  45

Gln Lys Glu Thr Thr Phe Tyr Thr Leu Val Cys Gly Leu Ala Val Thr
    50                  55                  60
Asp Leu Leu Gly Thr Leu Leu Val Ser Pro Val Thr Ile Ala Thr Tyr
65                  70                  75                  80
Met Lys Gly Gln Trp Pro Gly Asp Gln Ala Leu Cys Asp Tyr Ser Thr
                85                  90                  95
Phe Ile Leu Leu Phe Phe Gly Leu Ser Gly Leu Ser Ile Ile Cys Ala
            100                 105                 110
Met Ser Ile Glu Arg Tyr Leu Ala Ile Asn His Ala Tyr Phe Tyr Ser
        115                 120                 125
His Tyr Val Asp Lys Arg Leu Ala Gly Leu Thr Leu Phe Ala Val Tyr
    130                 135                 140
Ala Ser Asn Val Leu Phe Cys Ala Leu Pro Asn Met Gly Leu Gly Arg
145                 150                 155                 160
Ser Glu Arg Gln Tyr Pro Gly Thr Trp Cys Phe Ile Asp Trp Thr Thr
                165                 170                 175
Asn Val Thr Ala Tyr Ala Ala Phe Ser Tyr Met Tyr Ala Gly Phe Ser
            180                 185                 190
Ser Phe Leu Ile Leu Ala Thr Val Leu Cys Asn Val Leu Val Cys Gly
        195                 200                 205
Ala Leu Leu Arg Met Leu Arg Gln Phe Met Arg Arg Thr Ser Leu Gly
    210                 215                 220
Thr Glu Gln His His Ala Ala Ala Ala Ala Ala Val Ala Ser Val Ala
225                 230                 235                 240
Cys Arg Gly His Ala Ala Ala Ser Pro Ala Leu Gln Arg Leu Ser Asp
                245                 250                 255
Phe Arg Arg Arg Arg Ser Phe Arg Arg Ile Ala Gly Ala Glu Ile Gln
            260                 265                 270
Met Val Ile Leu Leu Ile Ala Thr Ser Leu Val Val Leu Ile Cys Ser
        275                 280                 285
Ile Pro Leu Val Val Arg Val Phe Ile Asn Gln Leu Tyr Gln Pro Ser
    290                 295                 300
Val Val Lys Asp Ile Ser Arg Asn Pro Asp Leu Gln Ala Ile Arg Ile
305                 310                 315                 320
Ala Ser Val Asn Pro Ile Leu Asp Pro Trp Ile Tyr Ile Leu Leu Arg
                325                 330                 335
Lys Thr Val Leu Ser Lys Ala Ile Glu Lys Ile Lys Cys Leu Phe Cys
            340                 345                 350
Arg Ile Gly Gly Ser Gly Arg Asp Gly Ser Ala Gln His Cys Ser Glu
        355                 360                 365
Ser Arg Arg Thr Ser Ser Ala Met Ser Gly His Ser Arg Ser Phe Leu
    370                 375                 380
Ser Arg Glu Leu Arg Glu Ile Ser Ser Thr Ser His Thr Leu Leu Tyr
385                 390                 395                 400
Leu Pro Asp Leu Thr Glu Ser Ser Leu Gly Gly Lys Asn Leu Leu Pro
                405                 410                 415
Gly Thr His Gly Met Gly Leu Thr Gln Ala Asp Thr Thr Ser Leu Arg
            420                 425                 430
Thr Leu Arg Ile Ser Glu Thr Ser Asp Ser Ser Gln Gly Gln Asp Ser
        435                 440                 445
Glu Ser Val Leu Leu Val Asp Glu Val Ser Gly Ser Gln Arg Glu Glu
    450                 455                 460
Pro Ala Ser Lys Gly Asn Ser Leu Gln Val Thr Phe Pro Ser Glu Thr
465                 470                 475                 480
Leu Lys Leu Ser Glu Lys Cys Ile
                485

(2) INFORMATION FOR SEQ ID NO: 6:

(i) SEQUENCE CHARACTERISTICS:
(A) LENGTH 1464 base pairs
(B) TYPE: nucleic acid
(C) STRANDEDNESS: single
(D) TOPOLOGY: linear
(ii) MOLECULE TYPE: cDNA to mRNA

(xi) SEQUENCE DESCRIPTION: SEQ ID NO: 6

ATGTCCATCC CCGGAGTCAA CGCGTCCTTC TCCTCCACTC CGGAGAGGTT GAACAGCCCA 60
GTGACCATTC CCGCAGTGAT GTTTATCTTC GGGGTGGTGG GCAACCTGGT GGCCATCGTA 120
GTATTGTGCA AGTCGCGCAA GGAGCAGAAG GAGACTACCT TTTACACTCT GGTATGTGGG 180
CTGGCTGTCA CTGACCTACT GGGCACATTG TTGGTAAGCC CAGTGACCAT CGCCACATAC 240
ATGAAGGGCC AGTGGCCCGG AGACCAGGCA TTGTGTGACT ACAGCACCTT CATCCTACTT 300
TTCTTCGGCC TGTCGGGTCT CAGCATCATC TGTGCCATGA GCATTGAGCG CTACCTGGCC 360
ATCAACCACG CCTACTTCTA CAGCCACTAC GTGGACAAGC GGCTGGCCGG TCTCACGCTC 420
TTCGCCGTCT ATGCATCTAA CGTGCTCTTC TGCGCACTGC CCAACATGGG CCTGGGTAGG 480
TCCGAGCGGC AGTACCCGGG GACCTGGTGC TTCATCGACT GGACCACCAA CGTAACGGCC 540
TACGCCGCCT TCTCTTACAT GTACGCGGGC TTCAGTTCCT TCCTCATCCT CGCCACCGTG 600
CTCTGCAATG TGCTGGTGTG CGGCGCGCTG CTCCGCATGC TCCGCCAGTT CATGCGCCGC 660
ACCTCGCTGG GCACGGAGCA GCACCACGCG GCCGCTGCAG CAGCGGTGGC TTCGGTGGCC 720
TGTCGGGGTC ACGCGGCCGC CTCCCCAGCC CTGCAGCGCC TCAGTGACTT TCGCCGCCGC 780
AGGAGCTTCC GGCGCATCGC GGGTGCAGAG ATCCAGATGG TCATCTTACT CATCGCCACC 840
TCTCTGGTGG TGCTCATCTG CTCCATTCCG CTCGTGGTGC GAGTGTTCAT CAACCAGTTA 900
TATCAGCCAA GTGTGGTGAA AGACATCAGC AGAAACCCGG ATTTGCAGGC CATCAGAATT 960
GCTTCTGTGA ACCCCATCCT GGACCCTTGG ATCTACATCC TTCTTCGGAA GACTGTGCTC 1020
AGTAAAGCCA TAGAAAAGAT CAAGTGCCTC TTCTGCCGCA TTGGTGGTTC TGGCAGAGAC 1080
GGTTCAGCAC AGCACTGCTC AGAGAGTCGG AGGACATCTT CTGCCATGTC TGGCCACTCC 1140
CGCTCCTTCC TCTCGCGGGA GTTGAGGGAG ATCAGCAGCA CCTCTCACAC CCTCCTATAC 1200
CTGCCAGACC TAACTGAAAG CAGCCTCGGA GGCAAGAATT TGCTTCCAGG TACGCATGGC 1260
ATGGGCCTGA CCCAAGCAGA CACCACCTCG CTGAGAACTT TGCGAATTTC AGAGACCTCA 1320
GACTCCTCCC AGGGCCAGGA CTCTGAGAGT GTCTTGTTGG TGGATGAGGT TAGTGGGAGC 1380
CAGAGAGAGG AGCCTGCCTC TAAGGGGAAC TCTCTGCAAG TCACGTTCCC CAGTGAAACG 1440
CTGAAATTAT CTGAAAAATG TATA 1464

(2) INFORMATION FOR SEQ ID NO: 7:

(i) SEQUENCE CHARACTERISTICS:
(A) LENGTH 1658 base pairs
(B) TYPE: nucleic acid
(C) STRANDEDNESS: single
(D) TOPOLOGY: linear
(ii) MOLECULE TYPE: cDNA to mRNA

(xi) SEQUENCE DESCRIPTION: SEQ ID NO: 7

CCGGTTCCGA GAGCGGCAAA GGCTTGACAA GTTCCGCACT GAGTGAGAAG AGACTGATGG 60
CTGAGGTTGG TGGTAGGTCC AGAACGACTG AGGCCTGAAC CGTGGGGCGC ACCCCACCCT 120
ACAGATACCA TCCCTAGATC GAACCGTGAG CTCCAAAGCT GTGTACTACT GACCACCATC 180
ATGTCCATCC CCGGAGTCAA CGCGTCCTTC TCCTCCACTC CGGAGAGGTT GAACAGCCCA 240
GTGACCATTC CCGCAGTGAT GTTTATCTTC GGGGTGGTGG GCAACCTGGT GGCCATCGTA 300
GTATTGTGCA AGTCGCGCAA GGAGCAGAAG GAGACTACCT TTTACACTCT GGTATGTGGG 360
CTGGCTGTCA CTGACCTACT GGGCACATTG TTGGTAAGCC CAGTGACCAT CGCCACATAC 420
ATGAAGGGCC AGTGGCCCGG AGACCAGGCA TTGTGTGACT ACAGCACCTT CATCCTACTT 480

TTCTTCGGCC TGTCGGGTCT CAGCATCATC TGTGCCATGA GCATTGAGCG CTACCTGGCC 540
ATCAACCACG CCTACTTCTA CAGCCACTAC GTGGACAAGC GGCTGGCCGG TCTCACGCTC 600
TTCGCCGTCT ATGCATCTAA CGTGCTCTTC TGCGCACTGC CCAACATGGG CCTGGGTAGG 660
TCCGAGCGGC AGTACCCGGG GACCTGGTGC TTCATCGACT GGACCACCAA CGTAACGGCC 720
TACGCCGCCT TCTCTTACAT GTACGCGGGC TTCAGTTCCT TCCTCATCCT CGCCACCGTG 780
CTCTGCAATG TGCTGGTGTG CGGCGCGCTG CTCCGCATGC TCCGCCAGTT CATGCGCCGC 840
ACCTCGCTGG GCACGGAGCA GCACCACGCG GCCGCTGCAG CAGCGGTGGC TTCGGTGGCC 900
TGTCGGGGTC ACGCGGCCGC CTCCCCAGCC CTGCAGCGCC TCAGTGACTT TCGCCGCCGC 960
AGGAGCTTCC GGCGCATCGC GGGTGCAGAG ATCCAGATGG TCATCTTACT CATCGCCACC 1020
TCTCTGGTGG TGCTCATCTG CTCCATTCCG CTCGTGGTGC GAGTGTTCAT CAACCAGTTA 1080
TATCAGCCAA GTGTGGTGAA AGACATCAGC AGAAACCCGG ATTTGCAGGC CATCAGAATT 1140
GCTTCTGTGA ACCCCATCCT GGACCCTTGG ATCTACATCC TTCTTCGGAA GACTGTGCTC 1200
AGTAAAGCCA TAGAAAAGAT CAAGTGCCTC TTCTGCCGCA TTGGTGGTTC TGGCAGAGAC 1260
GGTTCAGCAC AGCACTGCTC AGAGAGTCGG AGGACATCTT CTGCCATGTC TGGCCACTCC 1320
CGCTCCTTCC TCTCGCGGGA GTTGAGGGAG ATCAGCAGCA CCTCTCACAC CCTCCTATAC 1380
CTGCCAGACC TAACTGAAAG CAGCCTCGGA GGCAAGAATT TGCTTCCAGG TACGCATGGC 1440
ATGGGCCTGA CCCAAGCAGA CACCACCTCG CTGAGAACTT TGCGAATTTC AGAGACCTCA 1500
GACTCCTCCC AGGGCCAGGA CTCTGAGAGT GTCTTGTTGG TGGATGAGGT TAGTGGGAGC 1560
CAGAGAGAGG AGCCTGCCTC TAAGGGGAAC TCTCTGCAAG TCACGTTCCC CAGTGAAACG 1620
CTGAAATTAT CTGAAAAATG TATATAGTAG CTTAAAGG 1658

(2) INFORMATION FOR SEQ ID NO: 8:

(i) SEQUENCE CHARACTERISTICS:
(A) LENGTH 1658 base pairs
(B) TYPE: nucleic acid
(C) STRANDEDNESS: single
(D) TOPOLOGY: linear
(ii) MOLECULE TYPE: cDNA to mRNA
(vi) ORIGINAL SOURCE
(A) ORGANISM: Rat
(F) TISSUE TYPE: lung
(ix) FEATURE
(A) NAME/KEY: CDP
(B) LOCATION: 181..1647
(C) IDENTIFICATION METHOD: P

(xi) SEQUENCE DESCRIPTION: SEQ ID NO: 8

CCGGTTCCGA GAGCGGCAAA GGCTTGACAA GTTCCGCACT GAGTGAGAAG AGACTGATGG 60
CTGAGGTTGG TGGTAGGTCC AGAACGACTG AGGCCTGAAC CGTGGGGCGC ACCCCACCCT 120
ACAGATACCA TCCCTAGATC GAACCGTGAG CTCCAAAGCT GTGTACTACT GACCACCATC 180
ATG TCC ATC CCC GGA GTC AAC GCG TCC TTC TCC TCC ACT CCG GAG AGG 228
Met Ser Ile Pro Gly Val Asn Ala Ser Phe Ser Ser Thr Pro Glu Arg
1               5                   10                  15
TTG AAC AGC CCA GTG ACC ATT CCC GCA GTG ATG TTT ATC TTC GGG GTG 276
Leu Asn Ser Pro Val Thr Ile Pro Ala Val Met Phe Ile Phe Gly Val
            20                  25                  30
GTG GGC AAC CTG GTG GCC ATC GTA GTA TTG TGC AAG TCG CGC AAG GAG 324
Val Gly Asn Leu Val Ala Ile Val Val Leu Cys Lys Ser Arg Lys Glu
        35                  40                  45
CAG AAG GAG ACT ACC TTT TAC ACT CTG GTA TGT GGG CTG GCT GTC ACT 372
Gln Lys Glu Thr Thr Phe Tyr Thr Leu Val Cys Gly Leu Ala Val Thr
    50                  55                  60
GAC CTA CTG GGC ACA TTG TTG GTA AGC CCA GTG ACC ATC GCC ACA TAC 420
Asp Leu Leu Gly Thr Leu Leu Val Ser Pro Val Thr Ile Ala Thr Tyr
65                  70                  75                  80

ATG AAG GGC CAG TGG CCC GGA GAC CAG GCA TTG TGT GAC TAC AGC ACC 468
Met Lys Gly Gln Trp Pro Gly Asp Gln Ala Leu Cys Asp Tyr Ser Thr
                85                  90                  95
TTC ATC CTA CTT TTC TTC GGC CTG TCG GGT CTC AGC ATC ATC TGT GCC 516
Phe Ile Leu Leu Phe Phe Gly Leu Ser Gly Leu Ser Ile Ile Cys Ala
            100                 105                 110
ATG AGC ATT GAG CGC TAC CTG GCC ATC AAC CAC GCC TAC TTC TAC AGC 564
Met Ser Ile Glu Arg Tyr Leu Ala Ile Asn His Ala Tyr Phe Tyr Ser
        115                 120                 125
CAC TAC GTG GAC AAG CGG CTG GCC GGT CTC ACG CTC TTC GCC GTC TAT 612
His Tyr Val Asp Lys Arg Leu Ala Gly Leu Thr Leu Phe Ala Val Tyr
    130                 135                 140
GCA TCT AAC GTG CTC TTC TGC GCA CTG CCC AAC ATG GGC CTG GGT AGG 660
Ala Ser Asn Val Leu Phe Cys Ala Leu Pro Asn Met Gly Leu Gly Arg
145                 150                 155                 160
TCC GAG CGG CAG TAC CCG GGG ACC TGG TGC TTC ATC GAC TGG ACC ACC 708
Ser Glu Arg Gln Tyr Pro Gly Thr Trp Cys Phe Ile Asp Trp Thr Thr
                165                 170                 175
AAC GTA ACG GCC TAC GCC GCC TTC TCT TAC ATG TAC GCG GGC TTC AGT 756
Asn Val Thr Ala Tyr Ala Ala Phe Ser Tyr Met Tyr Ala Gly Phe Ser
            180                 185                 190
TCC TTC CTC ATC CTC GCC ACC GTG CTC TGC AAT GTG CTG GTG TGC GGC 804
Ser Phe Leu Ile Leu Ala Thr Val Leu Cys Asn Val Leu Val Cys Gly
        195                 200                 205
GCG CTG CTC CGC ATG CTC CGC CAG TTC ATG CGC CGC ACC TCG CTG GGC 852
Ala Leu Leu Arg Met Leu Arg Gln Phe Met Arg Arg Thr Ser Leu Gly
    210                 215                 220
ACG GAG CAG CAC CAC GCG GCC GCT GCA GCA GCG GTG GCT TCG GTG GCC 900
Thr Glu Gln His His Ala Ala Ala Ala Ala Ala Val Ala Ser Val Ala
225                 230                 235                 240
TGT CGG GGT CAC GCG GCC GCC TCC CCA GCC CTG CAG CGC CTC AGT GAC 948
Cys Arg Gly His Ala Ala Ala Ser Pro Ala Leu Gln Arg Leu Ser Asp
                245                 250                 255
TTT CGC CGC CGC AGG AGC TTC CGG CGC ATC GCG GGT GCA GAG ATC CAG 996
Phe Arg Arg Arg Arg Ser Phe Arg Arg Ile Ala Gly Ala Glu Ile Gln
            260                 265                 270
ATG GTC ATC TTA CTC ATC GCC ACC TCT CTG GTG GTG CTC ATC TGC TCC 1044
Met Val Ile Leu Leu Ile Ala Thr Ser Leu Val Val Leu Ile Cys Ser
        275                 280                 285
ATT CCG CTC GTG GTG CGA GTG TTC ATC AAC CAG TTA TAT CAG CCA AGT 1092
Ile Pro Leu Val Val Arg Val Phe Ile Asn Gln Leu Tyr Gln Pro Ser
    290                 295                 300
GTG GTG AAA GAC ATC AGC AGA AAC CCG GAT TTG CAG GCC ATC AGA ATT 1140
Val Val Lys Asp Ile Ser Arg Asn Pro Asp Leu Gln Ala Ile Arg Ile
305                 310                 315                 320
GCT TCT GTG AAC CCC ATC CTG GAC CCT TGG ATC TAC ATC CTT CTT CGG 1188
Ala Ser Val Asn Pro Ile Leu Asp Pro Trp Ile Tyr Ile Leu Leu Arg
                325                 330                 335
AAG ACT GTG CTC AGT AAA GCC ATA GAA AAG ATC AAG TGC CTC TTC TGC 1236
Lys Thr Val Leu Ser Lys Ala Ile Glu Lys Ile Lys Cys Leu Phe Cys
            340                 345                 350
CGC ATT GGT GGT TCT GGC AGA GAC GGT TCA GCA CAG CAC TGC TCA GAG 1284
Arg Ile Gly Gly Ser Gly Arg Asp Gly Ser Ala Gln His Cys Ser Glu
        355                 360                 365
AGT CGG AGG ACA TCT TCT GCC ATG TCT GGC CAC TCC CGC TCC TTC CTC 1332
Ser Arg Arg Thr Ser Ser Ala Met Ser Gly His Ser Arg Ser Phe Leu
    370                 375                 380
TCG CGG GAG TTG AGG GAG ATC AGC AGC ACC TCT CAC ACC CTC CTA TAC 1380

Ser Arg Glu Leu Arg Glu Ile Ser Ser Thr Ser His Thr Leu Leu Tyr
385                 390                 395                 400
CTG CCA GAC CTA ACT GAA AGC AGC CTC GGA GGC AAG AAT TTG CTT CCA    1428
Leu Pro Asp Leu Thr Glu Ser Ser Leu Gly Gly Lys Asn Leu Leu Pro
                405                 410                 415
GGT ACG CAT GGC ATG GGC CTG ACC CAA GCA GAC ACC ACC TCG CTG AGA    1476
Gly Thr His Gly Met Gly Leu Thr Gln Ala Asp Thr Thr Ser Leu Arg
            420                 425                 430
ACT TTG CGA ATT TCA GAG ACC TCA GAC TCC TCC CAG GGC CAG GAC TCT    1524
Thr Leu Arg Ile Ser Glu Thr Ser Asp Ser Ser Gln Gly Gln Asp Ser
        435                 440                 445
GAG AGT GTC TTG TTG GTG GAT GAG GTT AGT GGG AGC CAG AGA GAG GAG    1572
Glu Ser Val Leu Leu Val Asp Glu Val Ser Gly Ser Gln Arg Glu Glu
    450                 455                 460
CCT GCC TCT AAG GGG AAC TCT CTG CAA GTC ACG TTC CCC AGT GAA ACG    1620
Pro Ala Ser Lys Gly Asn Ser Leu Gln Val Thr Phe Pro Ser Glu Thr
465                 470                 475                 480
CTG AAA TTA TCT GAA AAA TGT ATA TAGTAGCTTA AAGG                    1658
Leu Lys Leu Ser Glu Lys Cys Ile
                485

## Description

### Field of the Invention

This invention relates to a Prostaglandin E (PGE) receptor. More particularly, it relates to a human or a rat PGE receptor polypeptides, a process for the preparation thereof, DNAs encoding the said polypeptides, a replication or a expression vector consisting of the said DNAs, a host cell transfected or transformed with the said vector, a monoclonal or polyclonal antibody against the said polypeptides, a pharmaceutical composition comprising the said polypeptides or antibody and a method of screening a compound possessing PGE agonistic or antagonistic activity by using the said polypeptide or the said host cell.

### Background

Prostanoids such as Prostaglandin (PG), Thromboxane (TX) and Leukotriene (LT) are a family of oxidized metabolites of arachidonic acid and exhibit various physiological activities in order to maintain local homeostasis in organisms (see The Pharmacological Basis of Therapeutics (Gilman, A. G., Goodman, L. S., Rall, T. W., and Murad, F., eds) 7th Ed., pp 660, Macmillan Publishing Co., New York (1985)). These physiological activities are controlled by a cell membrane receptor specific for each prostanoid (see Annu. Rev. Pharm. Tox., 10, 213 (1989) and Prostanoids and their Receptors. In Comprehensive Medicinal Chemistry., pp 643 (1990), Pergamon Press, Oxford).

Prostaglandin E (PGE) which is one of prostanoids, especially, Prostaglandin E₂ (PGE₂) is involved in contraction and relaxation of the digestive canal, gastric acid secretion, relaxation of smooth muscle and release of neurotransmitters etc. From the analysis of the physiological and pharmacological activity of PGE₂ and its acting site, it is thought that there are three subtypes of PGE₂ receptor i.e. EP1, EP2 and EP3 and that each relates to a different intracellular signal transduction. It is regarded that these receptor subtypes may induce the activation of phospholipase C and the stimulation or inhibition of adenylate cyclase (see Annu. Rev. Pharm. Tox., 10, 213 (1989) and Prostanoids and their Receptors. In Comprehensive Medicinal Chemistry. pp 643 (1990), Pergamon Press, Oxford).

One of them, EP2 receptor is thought to be involved in relaxation of smooth muscle of bronchus or circular smooth muscle of ileum or dilatation of various blood vessel and reabsorption of sodium and water in renal vessel (see Br. J. Pharmacol., 87, 45 (1986); Br. J. Pharmacol., 105, 271 (1992); J. Clin. Invest., 47, 1154 (1968) and J. Biol. Chem., 263, 6155 (1988)). In addition, the important action of PGE₂ through the EP2 receptor is the suppression of immune system and inflammation (see Am. Rev. Respir. Dis., 135 72 (1987)). Further, the action of PGE₂ through the EP3 receptor is regarded as useful for prevention and/or treatment of, for example, diarrhea, digestive ulcer, hypertension, asthma, immune disease or inflammation. In order to clarify these points, it is regarded as necessary to analyze the structure, signaling and distribution of the EP2 receptor.

### The disclosure of the invention

Recently, the present inventors have succeeded in cloning cDNAs for three subtypes of mouse PGE receptors, i.e., EP1, EP2 and EP3 (see Biochem. Biophys. Res. Commun., 184, 1197 (1992); J. Biol. Chem., 268, 20175 (1993); J. Biol. Chem., 268, 7759 (1993) and J. Biol. Chem., 267, 6463 (1992)). Further, in the specification of Japanese Patent Application Kokai Hei 5-320199 in which some of the inventors of the present invention were listed as inventors, a mouse PGE receptor as well as a human one were mentioned. However, as for a human PGE receptor, no specific sequence analysis has yet been carried out. Neither nucleotide nor amino acid sequences were shown.

Furthermore, in Biochem. Biophys. Res. Commun., 197, 263, (1993) by An *et al*., there was description about cloning a cDNA for a human EP2 receptor and its expression. The predicted amino acid sequence was shown. In addition, the corresponding nucleotide sequence has been deposited as accession No. L25124 in GenBank. However, the detailed investigation has proved that there are eight differences in the nucleotide sequence and three differences in the amino acid sequence between human EP2 receptor of the present invention and that of An *et al*.

By using a cDNA encoding a mouse EP2 receptor or its fragment as a probe, the present inventors have carried out the homology search in a human and a rat cDNAs libraries. From the results of sequencing isolated clones and expressing them, clones encoding a human and a rat EP2 receptor have been obtained. Thus, the present invention has been completed.

There were no polypeptides having the amino acid sequence which was identical to that of the polypeptide of the present invention, when the amino acid sequences of the polypeptides were compared by a computer to all known sequences in data base of Swiss Prot (Swiss Prot Release 2.0). Furthermore, there was no nucleotides sequence which were identical to that encoding the polypeptides of the present invention, when the nucleotide sequence was compared by a computer to all known sequences in data base of GenBank (GenBank Release 70.0). Therefore, it has been confirmed that the polypeptides of the present invention are entirely novel ones.

That is to say, the present invention relates to
1) a human EP2 receptor in substantially purified form,
2) a receptor described in the above 1), which is a polypeptide comprising an amino acid sequence shown in Seq. ID No. 1 or homologue thereof, or a fragment of the said sequence or homologue of the said fragment,
3) a polypeptide described in the above 2), consisting of the amino acid sequence shown in Seq. ID No. 1,
4) a DNA encoding the polypeptide described in the above 1), 2) or 3).
5) a DNA described in the above 4), having the nucleotide sequence shown in Seq. ID No. 2 or a fragment capable of hybridizing selectively to the said sequence,
6) a DNA described in the above 4), having the nucleotide sequence shown in Seq. ID No. 3 or a fragment capable of hybridizing selectively to the said sequence,
7) a rat EP2 receptor in substantially purified form,
8) a receptor described in the above 7), which is a polypeptide comprising an amino acid sequence shown in Seq. ID No. 5 or homologue thereof, or a fragment of the said sequence or homologue of the said fragment,
9) a polypeptide described in the above 8), consisting of the amino acid sequence shown in Seq. ID No. 5,
10) a DNA encoding the polypeptide described in the above 7), 8) or 9),
11) a DNA described in the above 10), having nucleotide sequence shown in Seq. ID No. 6 or a fragment capable of hybridizing selectively to the said sequence,
12) a DNA described in the above 10), having nucleotide sequence shown in Seq. ID No. 7 or a fragment capable of hybridizing selectively to the said sequence,
13) a replication or expression vector consisting of DNA described in the above 4) to 6) or 10) to 12),
14) a host cell transformed or transfected with a replication or expression vector described in the above 13),
15) a process for the preparation of a polypeptide described in the above 1) to 3) or 7) to 9), which consists of culturing a host cell described in the above 14) under the condition effective to express the said polypeptide,
16) a monoclonal or polyclonal antibody against a polypeptide described in the above 1) to 3) or 7) to 9),
17) a pharmaceutical composition which is characterized by comprising a polypeptide described in the above 1) to 3) or 7) to 9), or an antibody described in the above 16) and a pharmaceutically acceptable excipient and/or carrier,
18) a method for screening a compound possessing EP2 agonistic or antagonistic activity which is characterized by using a polypeptide described in the above 1) to 3) or 7) to 9),
19) a method for screening described in the above 18) which is characterized by using a host cell described in the above 14).

### Brief description of the drawing

Figure 1 showed the inhibitory activities of various ligands (PGE₂, PGE₁, iloprost (stable PGI₂ agonist), PGF₂α and PGD₂) on binding of [³H]PGE₂ onto the membrane of the cell transfected with phEPR1.

Figure 2 showed the inhibitory activities of various ligands (misoprostol (agonist for both EP2 and EP3), M&B28,767 (EP3 agonist), sulprostone (agonist for both EP1 and EP3) and butaprost (EP2 agonist)) on binding of [³H]PGE₂ onto the membrane of the cell transfected with phEPR1.

### Detailed description of the invention

The present invention relates to a human and a rat EP2 receptors in substantially purified form. Specifically with respect to a human EP2 receptor, the present invention relates to a polypeptide comprising an amino acid sequence shown in Seq. ID No. 1. In addition, the present invention relates to DNAs encoding these polypeptides. More specifically, the present invention relates to DNAs having the nucleotide sequence shown in Seq. ID No. 2 or 3.

With respect to a rat EP2 receptor, the present invention relates to a polypeptide comprising an amino acid sequence shown in Seq. ID No. 5 or homologue thereof, or a fragment of the said sequence or homologue of the said fragment; and DNAs encoding these polypeptides, more specifically, DNAs having nucleotide sequence shown in Seq. ID No. 6 or 7 or a DNA containing a fragment capable of hybridizing selectively to the nucleotide sequence shown in Seq. ID No. 6 or 7.

More particularly, the present invention relates to
(1) polypeptides comprising the amino acid sequences shown in Seq. ID No. 1 or 5,
(2) DNAs encoding the polypeptides described in the above (1),
(3) DNAs having nucleotide sequence shown in Seq. ID No. 2 or 6 and
(4) DNAs having nucleotide sequence shown in Seq. ID No. 3 or 7.

The polypeptides comprising the amino acid sequences shown in Seq. ID No. 1 or 5 mean not only those consisting of the amino acid sequence shown in Seq. ID No. 1 or 5, but also those with addition of polypeptides comprising amino acid sequence 20% or less, more preferable 5% or less numbers of total amino acids of polypeptides shown in Seq. ID No. 1 or 5 at N- and/or C-terminal of the said polypeptides.

The polypeptides comprising the amino acid sequences shown in Seq. ID No. 1 or 5 in purified form mean generally the polypeptide in a preparation in which 90% or more, e.g. 95%, 98% or 99% of the polypeptide in the preparation is that of the Seq. ID No. 1 or 5.

Homologues of the polypeptides comprising the amino acid sequence shown in Seq. ID No. 5 will be generally at least 70%, preferably at least 80 or 90% and more preferably at least 95% homologous to the polypeptides of Seq. ID No. 5 over a region of at least 100, preferably at least 150, for example 200, 250 or 300 contiguous amino acids. Such homologues are to be included in the definition of the polypeptide of the present invention.

In addition, fragments of the polypeptides comprising the amino acid sequences shown in Seq. ID No. 5 or a fragment of homologues of the said polypeptide mean at least 10, preferably at least 15, for example 20, 25, 30, 40, 50 or 60 amino acids in length.

DNAs capable of hybridizing selectively to the DNAs having the nucleotide sequence shown in Seq. ID No. 6 or 7 will be generally at least 70%, preferably at least 80 or 90% and more preferably at least 95% homologous to the said DNA over a region of at least 100, preferably at least 150, for example 200, 250 or 300 contiguous nucleotides. Such DNAs are to be included in the definition of the DNA of the present invention.

Fragments of the DNAs comprising the nucleotide sequence shown in Seq. ID No. 6 or 7 will be at least 10, preferably at least 15, for example 20, 25, 30 or 40 nucleotides in length, and are also included in the present invention.

It is possible to prepare a DNA of the present invention according to genetic engineering, chemical synthesis or methods known to a person skilled in the art.

A further embodiment of the invention provides replication and expression vectors consisting of DNA of the present invention. The vectors may be, for example, plasmid, virus or phage vectors provided with an origin of replication, optionally a promoter for the expression of the said DNA and optionally a regulator of the promoter. The vector may contain one or more selectable marker genes, for example an ampicillin resistance gene.

A further embodiment of the invention provides host cells transformed or transfected with the vectors for the replication and expression of DNA of the present invention, including the DNA comprising the nucleotide sequence shown in Seq. ID No. 2, 3, 6 or 7 or the open reading frame thereof. The cells may for example be bacterial, yeast, insect cell or mammalian cell.

A further embodiment of the invention provides a method of preparation of a polypeptide of the present invention which consists of culturing host cells of the present invention under conditions effective to express a polypeptide of the invention.

The polypeptide of the present invention includes one in which a part of the amino acid sequence shown in Seq. ID No. 5 is lacking (e.g., a polypeptide consisting of only essential sequence required for biological activity in mature protein), one in which a part of the amino acid sequence is replaced by other amino acids (e.g., those replaced by an amino acid having a similar property) and one in which other amino acids are added or inserted into a part of their amino acid sequence, as well as ones having the amino acid sequence shown in Seq. ID No. 1 or 5.

As is well known, there are from one to six kinds of codon encoding one amino acid (for example, one kind of codon for methionine (Met), and six kinds of codon for leucine (Leu)). Accordingly, the nucleotide sequence of DNA can be changed in order to encode the polypeptide having the same amino acid sequence.

The DNAs of the present invention, specified in (2) includes a group of every nucleotide sequences encoding polypeptides shown in Seq. ID No. 1 or 5. There is a probability of improving a yield of production of a polypeptide by changing a nucleotide sequence.

The DNAs specified in (3) are the embodiment of DNA shown in (2), and are sequence in the natural form.

The DNAs shown in (4) indicate the sequence of the DNAs specified in (3) with a untranslated region.

The DNAs having a nucleotide sequence shown in Seq. ID No. 3 or 7 may be prepared according to the following methods, that is:
(i) by isolating mRNA from a cell which produces the polypeptide of the present invention,
(ii) by preparing a first strand (single strand cDNA) from mRNA thus obtained, followed by preparing a second strand (double strand cDNA) (synthesis of cDNA),
(iii) by inserting cDNA thus obtained into a proper plasmid vector,
(iv) by transfecting recombinant vector into host cells (construction of cDNA library),
(v) by isolating a plasmid comprising the desired DNA with plaque hybridization from a cDNA library thus obtained,
(vi) by determining nucleotide sequence of the desired DNA.

Explained in detail, step (i) may be carried out in accordance with the method of Okayama, H *et al* (described in Method in Enzymology, 154, 3, (1987)) or the method of Chirgwin, J. M. *et al* (described in Biochem., 18, 5294 (1979)) from tissues in which EP2 receptors may be expressed in human or rat, preferably cells of tissue or cells of, for example, the lung, blood vessel, uterine or ileum.

Step (ii), (iii) and (iv) are a series of steps for preparing a cDNA library, and may be carried out in accordance with the method of Glubler & Hoffman (described in Gene, 25, 263, (1983)) with a slight modification.

As examples of the plasmid vector used in the step (iii), many that function in E.coli strains (e.g. pBR322) and in Bacillus subtilis (e.g. pUB110) are known, and l-ZAPII functioning in an E.coli strain is preferably used.

As for host cell used in step (iv), many are known. Any host cells can be used and DH5 competent cell (prepared according to the method described in Gene, 96, 23 (1990)) can be preferably used.

Recently, cDNA libraries of various tissues of each animal have been marketed. For example, a cDNA library derived from a human and a rat lung has been marketed from Clontech Co. These marketed cDNA libraries may be also preferably used.

Step (v) is itself a known method, for example, this step may be carried out by, for example, the plaque hybridization method or the colony hybridization method (described in Gene, 10, 63 (1980)) etc. As for an adequate probe, a DNA for a EP2 receptor of an other species of animal, a fragment thereof or a DNA having homology to the said DNA can be used.

Step (vi) is itself a known method, for example this step may be carried out by the dideoxy termination method or Maxam-Gilbert method.

Once the nucleotide sequences shown in Seq. ID No. 2, 3, 6 or 7 are determined, DNA of the present invention may be obtained by chemical synthesis, by PCR method or by hybridization with a fragment of the said nucleotide sequence, as a probe. Furthermore, DNA of the present invention may be obtained in a desired amount by transforming with a vector DNA containing a DNA of the present invention into a proper host, followed by culturing the transformant.

The polypeptides of the present invention (Seq. ID No. 1 or 5) may be prepared by:
(1) isolating and purifying from an organism or a cultured cell,
(2) chemically synthesizing, or
(3) using a skill of genetic engineering etc.,
preferably, by the method described in (3).

Examples of expression system (host cell-vector system) to prepare a polypeptide by using genetic engineering, are, for example, the expression system of bacteria, yeast, insect cell and mammalian cell.

For example, the expression in E.coli may be carried out by ligating a DNA encoding the mature protein (e.g., DNA encoding nucleotide sequence shown in Seq. ID No. 2 or 6) thus obtained to the downstream of a suitable promoter (e.g., trp promoter, lac promoter, lPL promoter, T7 promoter etc.), and then inserting it into a vector (e.g., pBR322, pUC18, pUC19 etc.) which functions in an E.coli strain to prepare an expression vector. And then, by culturing the E.coli (e.g., E.coli DH1, E.coli JM109, E.coli HB101) transfected with the expression vector thus obtained in a suitable medium, the desired polypeptides may be prepared from the obtained bacteria. When a bacterial signal peptide (e.g., signal peptide of pel B) is utilized, the desired polypeptide may be also produced in periplasm. Furthermore, a fusion protein with other polypeptide may be also produced easily.

Furthermore, expression in a mammalian cell may be carried out, for example, by inserting the DNA encoding the nucleotide sequence shown in Seq. ID No. 3 or 7 into the downstream of a suitable promoter (e.g., SV40 promoter, LTR promoter, metallothionein promoter) in a suitable vector (e.g., retrovirus vector, papilloma virus vector, vaccinia virus vector, SV40 vector) to obtain an expression vector, and transfecting a suitable mammalian cell (e.g., monkey COS-7 cell, Chinese hamster CHO cell, mouse L cell etc.) with the expression vector thus obtained, and then culturing the transformant in a suitable medium to get a desired polypeptide in the culture medium. The polypeptide thus obtained may be isolated and purified by conventional biochemical methods.

### Effect of the invention

The polypeptides themselves of the present invention can bind to PGE₂ specifically, so they may be used as agents for preventing or treating the diseases caused by excessive production of PGE₂, for example, diseases such as inflammation. They may be also used in screening a compound which can agonize or antagonize for EP2.

Further, polyclonal or monoclonal antibodies against the said polypeptide of the present invention can be used in the determination of the amount of the said polypeptide in organism, and thereby, may be utilized for the purpose of investigating the relationship between the said polypeptide and diseases, or for the purpose of diagnosing diseases, and the like. Polyclonal and monoclonal antibody thereof may be prepared by conventional methods by using the said polypeptide or the fragment thereof as an antigen.

DNA of the present invention may also be inserted into the vectors described above in an antisense orientation in order to provide for the production of antisense RNA. Such an antisense RNA may be used in a method of controlling the levels of a polypeptide of the invention in a cell.

The present invention also provides pharmaceutical compositions comprising a polypeptide of the invention, or an antibody thereof, in association with a pharmaceutically acceptable excipient and/or carrier.

The DNA of the present invention may be utilized as an important and essential template in preparing the polypeptide of the present invention which is expected to possess various use or for diagnosis of and in the treatment of genetic diseases. Further, genomic DNA may be isolated by using the DNA of the present invention as a probe. Similarly, it is possible to isolate genes having high homology to the DNA of the present invention in human or those of other species.

For the treatment etc. of inflammatory disease, the polypeptides of the present invention may be normally administered systematically or partially, usually by oral or parenteral administration, preferably orally, intravenously or intraventricularly.

The doses to be administered are determined depending upon age, body weight, symptom, the desired therapeutic effect, the route of administration, and the duration of the treatment etc. In the human adult, the doses per person per dose are generally between 100 mg and 100 mg, by oral administration, up to several times per day, and between 10 mg and 100 mg, by parenteral administration up to several times per day.

As mentioned above, the doses to be used depend upon various conditions. Therefore, there are cases in which doses lower than or greater than the ranges specified above may be used.

On administration of the compounds of the present invention, it may be used as solid compositions, liquid compositions or other compositions for oral administration, as, for example, injections, liniments or suppositories for parenteral administration.

Solid compositions for oral administration include compressed tablets, pills, capsules, dispersible powders and granules etc. Capsules include soft capsules and hard capsules.

In such compositions, one or more of the active compound(s) is or are admixed with at least one inert diluent (such as lactose, mannitol, glucose, hydroxypropyl cellulose, microcrystalline cellulose, starch, polyvinylpyrrolidone, magnesium metasilicate aluminate). The compositions may also comprise, as is normal practice, additional substances other than inert diluents: e.g. lubricating agents (such as magnesium stearate), disintegrating agents (such as cellulose calcium glycolate), stabilizing agents (such as human serum albumin, lactose), and assisting agents for dissolving (such as arginine, asparaginic acid).

The tablets or pills may, if desired, be coated with a film of gastric or enteric material such as sugar, gelatin, hydroxypropyl cellulose or hydroxypropylmethyl cellulose phthalate, etc., or be coated with two or more films. And further, coating may include containment within capsules of absorbable materials such as gelatin.

Liquid compositions for oral administration include pharmaceutically-acceptable emulsions, solutions, suspensions, syrups and elixirs etc. and may also contain inert diluent(s) commonly used in the art (for example, purified water, ethanol). Besides inert diluents, such compositions may also comprise adjuvants such as wetting agents, suspending agents, sweetening agents, flavouring agents, perfuming agents and preserving agents.

Other compositions for oral administration include spray compositions which may be prepared by known methods and which comprise one or more of the active compound(s). Spray compositions may comprise additional substances other than inert diluents: e.g. stabilizing agents such as sodium hydrogen sulfate, stabilizing agents to give the title compound isotonicity, isotonic buffer such as sodium chloride, sodium citrate and citric acid. For preparation of such spray compositions, for example, the method described in the United States Patent No. 2868691 or 3095355 may be used.

Injections for parenteral administration include sterile aqueous or non-aqueous solutions, suspensions and emulsions. As for aqueous solutions or non-aqueous solutions or suspensions, one or more active compound(s) is (are) admixed with at least one inert diluent. Aqueous diluents include distilled water for injection and physiological salt solution. Non-aqueous diluents include propylene glycol, polyethylene glycol, plant oil such as olive oil, alcohol such as ethanol, POLYSOLBATE80 (registered trade mark). Injections may comprise additional other than inert diluents: e.g. preserving agents, wetting agents, emulsifying agents, dispersing agents, stabilizing agent (such as human serum albumin, lactose), and assisting agents such as assisting agents for dissolving (for example, arginine, asparaginic acid).

They may be sterilized for example, by filtration through a bacteria-retaining filter, by incorporation of sterilizing agents in the compositions or by irradiation. They may also be manufactured in the form of sterile solid compositions, (for example, by freeze-drying), and which can be dissolved in sterile water or some other sterile diluents for injection immediately before use.

Other compositions for parenteral administration include liquids for external use, ointment, endemic liniments, suppositories for rectal administration and pessaries which comprise one or more of the active compound(s) and may be prepared by known methods.

### Best Mode to practice the invention

The following examples are illustrated, but not limit, the present invention.

### Example 1 :

### Cloning of cDNA for a human EP2 receptor

6 x 10⁵ of clones in a cDNA library (Clontech Code No.: CHAL 1033a) derived from human lung were transferred to nitrocellulosemembrane (marketed from Schleicher & Schuell) and screened by cross-hybridization using the cDNA for a mouse EP2 receptor and the fragment (about 1.6 kb) (described in J. Biol. Chem., 268, 7759 (1993)) prepared by digesting MP412 with SacI.

Hybridization was carried out at 37°C in 5 x SSPE (0.75 M NaCl, 0.05 M NaH₂PO₄· H₂O, 0.005 M EDTA.) containing 25% fromamide, 1 x Denhardt's solution, 0.1% SDS, 100 µg/ml heat-denatured salmon sperm DNA and a probe radiolabeled with ³²P for 20 hours. After hybridization, filters were washed twice at room temperature in 2 x SSC for 10 minutes. A number of positive clones were picked up, subcloned into pBluescript KS (-) (marketed from Invitrogen Co., Ltd.) and used for sequence analysis. Nucleotide sequences were determined on double strands by dideoxy chain termination method. From the result of the analysis, one representative clone, phEPR1, was full length cDNA clone having open reading frame (shown in Seq. ID No. 2) of 1464 bp. Full length nucleotide sequence was shown in Seq. ID No. 3. Predicted amino acid sequence encoding the open reading frame was shown in Seq. ID No. 1. Nucleotide sequence of phEPR1 as shown in Seq. ID No. 3 and predicted amino acid sequence were shown together in Seq. ID No. 4.

The nucleotide sequence and amino acid sequence of a human EP2 receptor obtained in the present invention were compared with those of reports by An *et al* (The right superscript number attached to each base and amino acid means the position of each one from "A" of ATG as expression starting codon and "Met" as expression starting amino acid, respectively.).

C⁻⁸⁴ in the nucleotide sequence of the present invention corresponds to "G" in the report by An *et al*. This is shown below as "C⁻⁸⁴ → G". Similarly, there were a total of eight differences (C⁻⁷⁶ → G, C⁻⁵⁶ → C, C⁻⁵⁵ → G, C³¹⁵ → T, A⁸⁷⁶ → G, GCT¹³⁸⁹GGG → GCTTGGG and GCC¹³⁹⁸CCT → GCCCT) between them. Further, from these differences of the nucleotide sequence, it has revealed that there were three differences in the amino acid sequence (Gly⁴⁶⁴ → Trp, Pro⁴⁶⁵ → Ala, Ala⁴⁶⁶ → Cys).

### Example 2

### Cloning a cDNA for a rat EP2 receptor

By using 3 x 10⁵ of clone in a cDNA library derived from rat lung (Clontech Code No. CLRL 1008a), cross-hybridization was carried out according to the procedure as Example 1. A number of positive clones were picked up, subcloned into pBluescript KS (-) (marketed from Invitrogen Co., Ltd.) and used for sequence analysis. Nucleotide sequences were determined on double strands by dideoxy chain termination method. From the result of the analysis, one representative clone, prEPR1, was a full length cDNA clone having an open reading frame (shown in Seq. ID No. 6) of 1464 bp. Full length Nucleotide sequence is shown in Seq. ID No. 7. Predicted amino acid sequence encoding the open reading frame is shown in Seq. ID No. 5. Nucleotide sequence of prEPR1 as shown in Seq. ID No. 7 and predicted amino acid sequence are shown together in Seq. ID No. 8.

### Example 3

### Expression in COS cells and ligand binding assay

The HindIII-Xba1 insert of cDNA of prEPR1 was subcloned into pRc/RSV which is a expression vector for eukaryotic cells. The resultant plasmid DNA was transfected into COS-7 cells by DEAE-dextran method (described in Mol. Cell. Biol., 4, 1641 (1984)). The transfected COS-7 cell clones were cultured for 72 hours, harvested and homogenized using a Potter-Elvehjem homogenizer in a solution consisting of 25 mM Tris-HCl (pH 7.5), 0.25 M sucrose, 10 mM MgCl₂, 1 mM EDTA and 0.1 mM phenylmethylsulfonylfluoride. The homogenate was centrifuged at 800 x g for 10 minutes. The supernatant was pooled and the pellet was resuspended in the solution consisting of 25 mM Tris-HCl (pH 7.5), 0.25 M sucrose, 10 mM MgCl₂, 1 mM EDTA and 0.1 mM phenylmethylsulfonylfluoride, homogenized as above, and centrifuged again. Thus obtained supernatant and the pooled supernatant were admixed and centrifuged at 100,000 x g for 30 minutes. The resultant pellet was suspended in 10 mM MES (2-(N-morpholino)ethane sulphonic acid monohydrate, pH 6.0), 10 mM MgCl₂ and 1 mM EDTA (the suspension buffer) and used as the crude membrane fraction.

[³H]PGE₂ binding assay was carried out as follows. That is to say, the crude membrane fraction, 60 µg protein and various concentrations of PGE₂ (for Scatchard analysis) or 4 nM PGE₂ (for displacement experiment) were suspended in the suspension buffer in total volume of 100 µg and incubated at 30°C for 1 hour. The incubation was terminated by the addition of 2 ml of the ice-chilled suspension buffer (10 mM KH₂PO₄, 10 ml MgCl₂ and 1 mM EDTA). The mixture was rapidly filtered through a Whatman GF/C filter. The filter was washed four times with 2 ml of the ice-chilled suspension buffer. The radioactivity on the filter was measured by the conventional methods.

This assay showed that [3H]PGE₂ which is a ligand for the PGE₂ receptor, bound specifically onto the cell membrane of COS-7 cell expressing prEPR1. Scatchard analysis of this binding showed a dissociation constant of 6.1 nM and maximum binding of 641 fmol/mg protein.

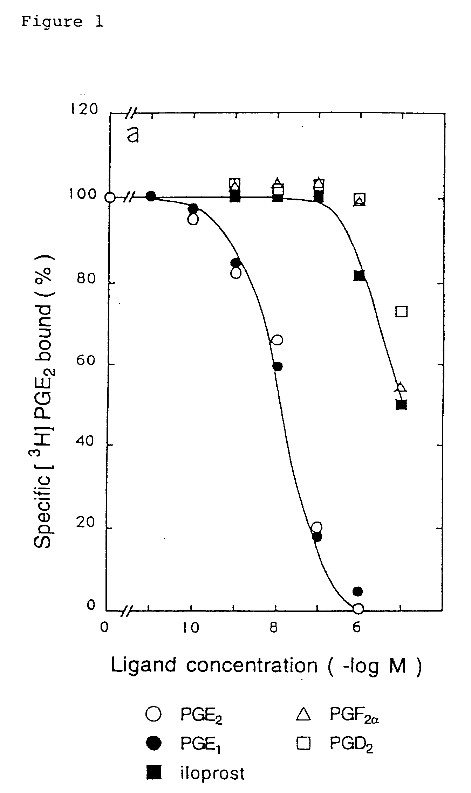

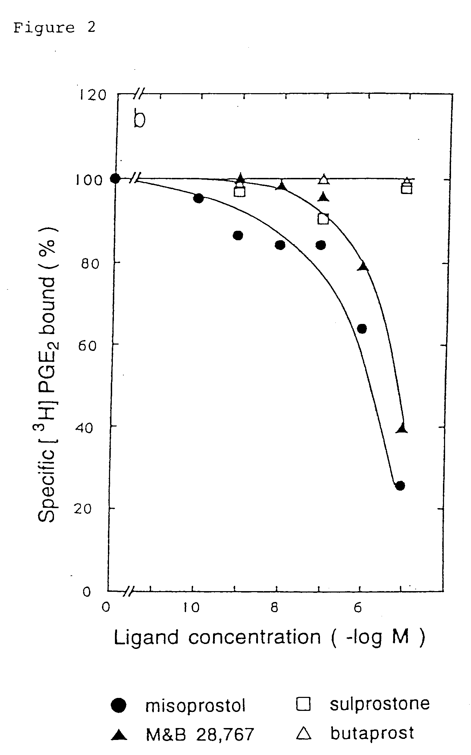

In addition, inhibitory activity of various prostanoids on specific [³H]PGE₂ binding was shown in Figure 1. Further, inhibitory activity of various EP agonists on specific [³H]PGE₂ binding was shown in Figure 2.

The rank order of inhibitory activity of various prostanoids is as PGE₂ = PGE₁ >> iloprost > PGF₂α= PGD₂. On the other hand, as for various EP agonists, misoprost (agonist for both EP2 and EP3) > M&B28,767 (EP3 agonist) can bind at rank order as shown, but sulprostone (agonist for both EP1 and EP3) and butaprost (EP2 agonist) can not bind. Butaprost can not bind to a mouse EP2 receptor either, so it is thought that there is a difference in ability to bind specifically.

## Claims

1. A human EP2 receptor in substantially purified form.

2. A receptor according to claim 1, which is a polypeptide comprising an amino acid sequence shown in Seq. ID No. 1 or homologue thereof, or a fragment of the said sequence or homologue of the said fragment.

3. A polypeptide according to claim 2, consisting of the amino acid sequence shown in Seq. ID No. 1.

4. A DNA encoding the polypeptide according to claim 1, 2 or 3.

5. A DNA according to claim 4, having the nucleotide sequence shown in Seq. ID No. 2 or a fragment capable of hybridizing selectively to the said sequence.

6. A DNA according to claim 4, having the nucleotide sequence shown in Seq. ID No. 3 or a fragment capable of hybridizing selectively to the said sequence.

7. A rat EP2 receptor in substantially purified form.

8. A receptor according to claim 7, which is a polypeptide comprising an amino acid sequence shown in Seq. ID No. 5 or homologue thereof, or a fragment of the said sequence or homologue of the said fragment.

9. A polypeptide according to claim 8, consisting of the amino acid sequence shown in Seq. ID No. 5.

10. A DNA encoding the polypeptide according to claim 7, 8 or 9.

11. A DNA according to claim 10, having nucleotide sequence shown in Seq. ID No. 6 or a fragment capable of hybridizing selectively to the said sequence.

12. A DNA according to claim 10, having nucleotide sequence shown in Seq. ID No. 7 or a fragment capable of hybridizing selectively to the said sequence.

13. A replication or expression vector consisting of DNA according to claim 4 to 6 or 10 to 12.

14. A host cell transformed or transfected with a replication or expression vector according to claim 13.

15. A process for the preparation of a polypeptide according to claim 1 to 3 or 7 to 9, which consists of culturing a host cell according to claim 14 under the condition effective to express the said polypeptide.

16. A monoclonal or polyclonal antibody against a polypeptide according to claim 1 to 3 or 7 to 9.

17. A pharmaceutical composition which is characterized by comprising a polypeptide according to claim 1 to 3 or 7 to 9, or an antibody according to claim 16 and a pharmaceutical acceptable excipient and/or carrier.

18. A method for screening a compound possessing EP2 agonistic or antagonistic activity which is characterized by using a polypeptide according to claim 1 to 3 or 7 to 9.

19. A method for screening according to claim 18 which is characterized by using a host cell according to claim 14.
